# EUROPEAN PATENT APPLICATION

(11) **EP 1 774 983 A1**
(43) Date of publication of application: **18.04.2007**
(21) Application number: 06122448.1
(22) Date of filing: 17.10.2006
(51) Int. Cl.: A61L 15/12

(54) **A binder composition**

(30) Priority: 17.10.2005 SE 0502287
(71) Applicant: Celanese Emulsions Norden AB, 284 21 Perstorp (SE)
(72) Inventor: Jönsson, Jan-Erik, SE-226 53, LUND (SE); Mauritsson, Jessica, SE-217 73, MALMÖ (SE); Stridfeldt, Chatrin, SE-436 58, HOVÅS (SE); Rosseland, Berit, SE-434 93, VALLDA (SE); Karlsson, Ola, SE-222 41, LUND (SE)
(74) Representative: Henriksson, Dan Ragnar Mikael

(57) **Abstract**

Provided is a new binder composition comprising a pH controlling agent and polymer dispersion. The binder provides antimicrobial properties to various kinds of materials, including those used in antibacterial packing material, hygiene and health care products. The binder composition also show satisfactory bonding properties, when the binder is applied on fibrous materials. Finally, the binder is easily applied, eg., through dipping, coating or spraying methods, onto the relevant surfaces or areas.

## Description

### Field of the invention

The invention relates to a new binder composition which is stable, provides antibacterial properties and bonding properties to fibrous materials for use in packaging, hygiene and health care products.

### Background of the invention

It is known that the presence of microorganisms may alter the end-use properties, appearance or odour of products possessing organic components, components which are able to offer some form of nutritional support to the growth of microorganisms. This is relevant for products used in direct contact with the body or skin. Such products include, for example, disposable nonwoven articles including diapers, sanitary napkins, antibacterial packaging material, surgical drapes, hospital pads and adult incontinence protection products.

Efforts have been made to create products with various forms of antimicrobial protection. Incorporation of a broad spectrum of antimicrobial, bacteriostatic or deodorizing agents into some part of the product is commonly used. For example, the agent(s) may be sprayed onto the transfer layer located next to the outermost skin contact layer, of an incontinence brief or napkin. (L. Baker, Nonwovens World, Oct-Nov 2002, pp. 78).

Another alternative is to incorporate the antimicrobial, bacteriostatic or deodorizing agent into the binder used in the assembly of the final product. However, care must be taken to ensure that the amount, type and form of antimicrobial agent used does not compromise the final bonding properties of the binder. Appropriate bonding strength must still exist to properly adhere the substrates involved in the products. In the article "A new Approach to Odor Control for Adult Incontinence" (L. Baker, Nonwovens World, Oct-Nov 2002, pp. 78), an approach to odour control for adult incontinence products is described, which include combined use of pH-modifiers and microbial agents.

Many antimicrobial compositions for use in e.g. nonwoven hygiene materials are hotmelt compositions, which preferably are solvent free, prepared by blending various composition components (e.g. a polymer, tackifying resin, plasticizer, wax, antioxidant) with a bacteriostatic agent. Examples are described in WO 0245763 and US 6503531.

Antimicrobial compositions in the form of dispersions can also be used for nonwoven materials. EP 1151665 relates to an dispersion comprising a deodorant and antimicrobial component, a binder resin and an aqueous or oily medium. The deodorant and antimicrobial component comprises an oxide or hydroxide of an alkaline earth metal, hydrated silica and a cationic surfactant.

Other compositions may contain an antimicrobial polymer giving the antibacterial properties. US 20030027889 relates to an antimicrobial paste glue, e.g. for wall coverings. In W003024494 the antimicrobial constituent contains metal silver particles.

In all the documents mentioned above an addition of a special antimicrobial, bacteriostatic and/or deodorizing agent was necessary. The special agent is meant to indicate an additive not usually included in the main product and which provides the antibacterial function. The polymer component is also often used in solid form and not in dispersion, since it is more difficult to formulate a dispersion with the desired properties.

The use of special antimicrobial, bacteriostatic and/or deodorizing agents make it more likely to show incompatibility problems in the final product, in this case a binder composition. Accordingly, the agents may affect the final bonding properties of the binder composition. Another issue to consider is the toxicity of such an agent. Because it is supposed to be used in hygiene products with close contact to the skin, toxic or allergic reactions due to the antimicrobial, bacteriostatic or deodorizing agent are not accepted, therefore limiting the choice of agents that can be used.

The use of polymers in the solid phase can give rise to formulation problems in the binder composition, due to the typically high viscosity of polymers above a certain molecular weight, which varies with the type of polymer, and the amount used in the formulation. High viscous binder systems give rise to application problems, e.g., they are not easily dipped, coated or sprayed onto the relevant surfaces or materials. These binder systems needs special application apparatus.

On the other hand, the use of polymers in a dispersion can give rise to other problems, like stability and compatibility problems in the binder composition. Different additives used to provide the desired properties of the composition might not be compatible, resulting in formulation difficulties.

A need exists for an improved stable binder composition system which can ensure both good bonding properties as well as the desired antimicrobial, bacteriostatic and/or deodorizing properties in the final end product.

There is also a need for an improved binder composition system which is easily applied to the surfaces or materials requiring antimicrobial or bacteriostatic protection.

### Summary of the invention

The present invention provides a new binder composition possessing the above mentioned desired properties. The binder composition can provide bacteriostatic properties to various kinds of fibrous materials and also have satisfactory bonding properties. The binder composition of the invention comprise a binder and a pH controlling agent which maintains the pH in an acidic range in ready-made material.

The binder composition may also include other additives that commonly are used within the field, like tackifying resins, surfactants, antioxidants, plasticizers, waxes, or other suitable additives normally used in a binder formulation.

In one aspect, the binder composition comprises a polymer dispersion as the binder.

In another aspect, the pH controlling agent maintains the pH in a range of 1-6, preferably 2-5, most preferred 3-4, in the binder composition.

In still another aspect, the pH controlling agent is a buffer or a (poly)carboxylic acid. The buffer is preferably selected from the group consisting of inorganic and organic buffers, and more preferably selected from the group consisting of phosphate-citrate buffer, citrate-citric acid buffer, phtalate-hydrochloric acid buffer.

In another aspect, the (poly)carboxylic acid is added as a polymer or co-polymerized as a monomer of carboxylic acid with the binder. The (poly)carboxylic acid is preferably selected from the group consisting of polyacids with a pKa below 6 of the invention suitable for addition to the dispersion to obtain the desired properties, and most preferred polyacrylic acid, polymethacrylic acid, polyitaconic acid.

In yet another aspect, the pH controlling agent is present in the binder composition in an amount of 0,1-10%, preferably 0.2-5%, most preferred 0.3-4%, by weight, based on dry matter.

In still another aspect, the binder is a polymer dispersion comprising a polymer derived from at least one member selected from the group consisting of ethylene and vinyl containing compounds, wherein the vinyl containing compounds include at least one unsaturated organic acid amide, at least one N-methylol derivative of at least one unsaturated organic acid amide and/or at least one ether of at least one N-methylol derivative of at least one unsaturated organic acid amide. Preferably the vinyl containing compounds are selected from the group consisting of acryl amide; N-methylolacrylamide; N-methylolmethacrylamide; N-(iso-butoxymethyl)acrylamide; N-(n-butoxymethyl)acrylamide; glycidyl acrylate; glycidyl methacrylate; allyl methacrylate; di, tri and multifunctional ester of a di, tri or polyhydric alcohol and acrylic and/or methacrylic acid; trialkoxyvinylsilane; alkyldialkoxyvinylsilane; acryloxyalkoxysilane; acryloxyalkylalkoxysilane; alkoxyacrylsilane; methacryloxyalkoxysilane; methacryloxyalkylalkoxysilane and/or alkoxymethacrylsilane. Preferably the di, tri and multifunctional ester of a di, tri or polyhydric alcohol and acrylic and/or methacrylic acid is butanediol diacrylate, dipropylene glycole diacrylate, hexanediol diacrylate, tripropylene glycol diacrylate, butanediol dimethacrylate, ethylene glycol dimethacrylate, diethylene glycol methacrylate, trimethylolpropane triacrylate, trimethylolpropane triacrylate, trimethylolpropane triacrylate, ethoxylated trimethylolpropane tri acrylate and/or ethoxylated pentaerythritol diacrylate. Preferably said alkyl and/or alkoxy compounds is linear or branched having 1-4 carbon atoms, and most preferably it is trimethoxyvinylsilane; triethoxyvinylsilane; triisopropoxyvinylsilane; methoxymethacrylsilane and/or 3-methacryloxypropyltriisopropoxysilane.

In still another aspect, the binder is a polymer dispersion comprising a polymer derived from at least one member selected from the group consisting of ethylene and vinyl esters, wherein the vinyl esters include vinyl acetate, vinyl propionate, vinyl butyrate, vinyl isobutyrate, vinyl 2-etylhexanoate, vinyl versatate, vinyl laurate.

In still another aspect, the binder is a polymer dispersion comprising a polymer derived from at least one member selected from the group consisting of ethylene and styrene acrylics, wherein the styrene acrylics include esters of acrylic and methacrylic acids.

Most preferred is that the main constituents in the polymer comprise vinyl acetate-co-ethylene.

Another object of the invention is to provide a polymerisation process yielding a binder composition, wherein the polymerisation process can be performed in the presence of a pH controlling agent. The pH controlling agent may be added before, during or after the polymerisation reaction.

Yet another object of the invention is the use of a binder composition for providing antibacterial properties in a fibrous material.

In one aspect, the binder composition is effective against different bacteria families, such as E. coli, P. mirabilis, E. faecalis.

Still another object of the invention is to provide a fibrous material treated with a binder composition, wherein the fibrous material comprise synthetic or natural fibers, or mixtures thereof. The fibrous material may comprise cellulosic fibers, and might be non-woven materials.

Another object of the invention is to provide fibrous material, wherein the binder is applied to the fibrous material by e.g. spraying, print bonding, saturation or impregnation.

Yet another object of the invention is to provide fibrous material, wherein the binder is applied to the fibrous material in an amount of 1-50%, preferably 3-30%, more preferably 5-25%, most preferably 7-20%, by weight.

The fibrous material may be used for manufacturing diapers, incontinence protection products, female hygiene products, antibacterial packing material, sanitary and medical articles.

### Detailed description of the invention

The present invention relates to new binder possessing antimicrobal properties, which may have applications in various kinds of materials, including those used in hygiene and health care products. The binder also show satisfactory bonding properties. Finally, the binder is easily applied, e.g., through dipping, coating or spraying methods, onto the relevant surfaces or areas. The binder composition is easily applied to the relevant surfaces or areas, e.g. a fibrous material, by spraying, dipping, coating, print bonding, saturation or impregnation, but it is not limited to these processes in any way.

More specifically, the binder composition comprise at least one pH controlling agent and a polymer dispersion.

The pH controlling agent in the binder composition functions as the active antimicrobal agent and is selected from the group consisting of inorganic and organic buffers, and (poly)carboxylic acids, copolymerised or added. If The pH controlling agent keep the binder within a pH-range of 1-6, preferably 2-5, and most preferably 3-4. All buffers or (poly)carboxylic acids effective within these pH-ranges can be used. Preferred buffers are phosphate-citrate buffer, citrate-citric acid buffer, phtalate-hydrochloric acid buffer, and particularly phosphate-citrate buffer is preferred. If a (poly)carboxylic acid is used it is either added as the polymer, polycarboxylic acid, to the binder or it is co-polymerized as the monomer, carboxylic acid, with the binder. Preferably polyacids with a pKa below 6 of the invention suitable for addition to the dispersion to obtain the desired properties are used, e.g. selected among polyacrylic acid, polymethacrylic acid, polyitaconic acid. The pH controlling agent is present in the binder composition in an amount of 0.1-10%, preferably 0.2-5%, but most preferably 0.3-4% by weight.

The polymer dispersion in the binder composition functions as an adhesive to the fibrous materials it is applied on, a medium for the pH controlling agent, and it comprises at least one polymer selected from the group consisting of vinyl acetate-co-ethylene, vinyl acetate, vinyl acetate copolymers (with acrylates), styrene acrylics or vinyl containing compounds. Vinyl containing compounds include unsaturated organic acid amide, N-methylol derivative of unsaturated organic acid amide and/or ether of N-methylol derivative of unsaturated organic acid amide, preferably the vinyl containing compounds are selected from the group consisting of acryl amide; N-methylolacrylamide, N-methylolmethacrylamide; N-(iso-butoxymethyl)acrylamide and/or N-(n-butoxymethyl)acrylamide; glycidyl acrylate; glycidyl methacrylate; allyl methacrylate; di, tri and multifunctional ester of a di, tri or polyhydric alcohol and acrylic and/or methacrylic acid; trialkoxyvinylsilane; alkyldialkoxyvinylsilane; acryloxyalkoxysilane; acryloxyalkylalkoxysilane; alkoxyacrylsilane; methacryloxyalkoxysilane; methacryloxyalkylalkoxysilane and/or alkoxymethacrylsilane. Preferably the di, tri and multifunctional ester of a di, tri or polyhydric alcohol and acrylic and/or methacrylic acid is butanediol diacrylate, dipropylene glycole diacrylate, hexanediol diacrylate, tripropylene glycol diacrylate, butanediol dimethacrylate, ethylene glycol dimethacrylate, diethylene glycol methacrylate, trimethylolpropane triacrylate, trimethylolpropane triacrylate, trimethylolpropane triacrylate, ethoxylated trimethylolpropane tri acrylate and/or ethoxylated pentaerythritol diacrylate. Preferably said alkyl and/or alkoxy compounds are linear or branched having 1-4 carbon atoms, and most preferably they are trimethoxyvinylsilane; triethoxyvinylsilane; triisopropoxyvinylsilane; methoxymethacrylsilane and/or 3-methacryloxypropyltriisopropoxysilane; and vinyl esters like vinyl propionate, vinyl isobutyrate, vinyl 2-etylhexanoate, vinyl versatate and/or vinyl laurate. The polymer dispersion preferably comprise as main constituents vinyl acetate-co-ethylene.

Other additives used in conventional amounts in the polymer dispersion are additives known in the art of polymerisation, to be able to produce a stable polymer dispersion. For example, surfactants, thickening agents, antioxidants, plasticizers and waxes.

The bacteria families against which the binder is effective include bacteria sensitive within the pH-range 1-6. Examples of bacteria that is affected is Escherichia coli, Proteus mirabilis and Enterococcus faecalis.

A fibrous material can be treated with a binder composition according to the invention, wherein the fibrous material comprise synthetic and/or natural fibers. The fibrous material may comprise cellulosic fibers, and might be non-woven materials.

The binder according to the invention can be applied to the fibrous material in an amount of 1-50%, preferably 3-30%, more preferably 5-25%, most preferably 7-20%, by weight. The fibrous material may then be used for manufacturing diapers, incontinence protection products, female hygiene products, antibacterial packing material, sanitary and medical articles.

### Examples

The examples given below illustrate the invention. These examples are present to exemplify the invention and are not intended to limit, in any way, the invention as covered by the accompanied claims.

### Experimental Procedures

### 1.1 Buffer preparation.

In order to prepare the buffers, requested amounts of A and B were dissolved in C and added to the dispersion during stirring. The amounts of A, B and C are presented in the tables. The final solids content of the pH buffered dispersion were 50%.

### 1.2 Preparation of bonded fibrous material.

Bonded fibrous material was prepared in two different ways, either through spraying or impregnating fibrous material with dispersion. The binder composition was adjusted to 10-15% add on (dry weight). The material was dried and cured at 160°C for 5 minutes.

### 1.3 Description of the methods used to determine the Elution pH.

10 ml test solution was added to 0.2 g fibrous material. After 30 minutes the solution was retrieved and the pH of the solution was measured. Pure test solution and test solution retrieved from untreated fibre were used as references. Dual tests were made for each sample. Composition of test solution: 0.9% NaCl in distilled water.

### 1.4 Description of the bacterial tests.

Preparation of samples (application of binder) was executed in the same way as in 1.2. The samples were thereafter sent to SIK (The Swedish Institute for Food and Biotechnology) who performed the bacterial tests, considering restraining microbial growth. After inoculation of bacteria (with urine) the samples were put in a heating chamber at 35 °C. The unit cfu in the Table refer to the number of bacterial colonies forming units and the sample judging was visually determined. Further it should also be notable that the scale is logarithmic.

### 2 Different Buffer systems

### 2.1 Citrate- Phosphate Buffer in a nonionic (NI) anionic (AI) surfactant stabilized vinyl acetate-co-ethylene (VA/E) copolymer dispersion. The effect on elution pH of different buffered pH's with varying amount of buffer.

| Buffer pH | Buffer formula | Buffer description | Amount buffer (g/100g 50% dispersion) | pH in dispersion | Elution pH |
|---|---|---|---|---|---|
| 2,6 | 34,4 wt-% A | 2,6a | 0,25 | 4,0 | 6,3 |
| | 15,6 wt-% B | 2,6b | 2,5 | 3, 0 | 5,2 |
| | 50 wt-% C | 2,6c | 6,25 | 2,8 | 4,2 |
| | | 2,6d | 12,5 | 2,6 | 3,8 |
| 3,6 | 24 wt-% A | 3,6a | 0,36 | 4,3 | 6,2 |
| | 26 wt-% B | 3, 6b | 3, 6 | 4, 0 | 5,4 |
| | 50 wt-% C | 3,6c | 9 | 3,9 | 4,8 |
| | | 3, 6d | 21,6 | 3,8 | 4,5 |
| 4,6 | 12,4 wt-% A | 4,6a | 0,43 | 4,5 | 5,6 |
| | 37,6 wt-% B | 4,6b | 4,27 | 4,7 | 5,4 |
| | 50 wt-% C | 4,6c | 10,67 | 4,7 | 5,3 |
| | | 4,6d | 21,34 | 4,7 | 5,3 |
| Dispersion without buffer | n.a. | n.a. | 0 | 4,4 | 5,5 |
| Base material | n.a. | n.a. | 0 | n.a. | 6,0 |

| | | | | | |
|---|---|---|---|---|---|
| A = citric acid B = dibasic sodium phosphate C = water | | | | | |

### Results 2.1

The results show that the buffered dispersions achieve a lower ph and, also, that the elution pH is lowered when higher amounts of buffer is used.

### 2.1.1 Citrate- Phosphate Buffer in vinyl acetate-co-ethylene (VA/E) copolymer dispersion with different stabilisation systems. The effect on elution pH of varying amounts of buffer and types of stabilization systems at buffer pH 3.6.

| Stabilisation system | Buffer pH | Amount buffer (g/100g 50% dispersion) | pH in dispersion | Elution pH |
|---|---|---|---|---|
| Surfactant (NI/AI) | 3, 6 | 9 | 3, 9 | 4,8 |
| | | 21,6 | 3,8 | 4,5 |
| | Dispersion without buffer | 0 | 4.4 | 5.5 |
| Poly vinylalcohol (PVOH) | 3,6 | 9 | 3,8 | 4,4 |
| | | 21,6 | 3,7 | 4,3 |
| | Dispersion without buffer | 0 | 4,0 | 5,4 |
| Hydroxy ethyl cellulose (HEC) | 3,6 | 9 | 3,7 | 4,3 |
| | | 21,6 | 3,7 | 4,2 |
| | Dispersion without buffer | 0 | 3,3 | 4,8 |
| | Base material | 0 | | 6,5 |

| | | | | |
|---|---|---|---|---|
| Buffer formula: 24 wt-% citric acid + 26 wt-% dibasic sodium phosphate + 50 wt-% water | | | | |

### Results 2.1.1

Same as above; the results show that the buffered dispersions achieve a lower ph and that also the elution pH is lowered when higher amounts of buffer is used. With the addition that the stabilization systems used here do not have an effect on the elution pH and also that the dispersions studied here are not affected in stability by the addition of rather large amounts of salts (buffer).

### 2.2 Phthalate-Hydrochloric Acid Buffer in a nonionic (NI) anionic (AI) surfactant stabilized vinyl acetate-co-ethylene (VA/E) copolymer dispersion. The effect on elution pH of varying amounts of buffer at buffer pH 3.6.

| Buffer pH | Buffer formula (amount/100 g 50 % dispersion) | pH in dispersion | Elution pH |
|---|---|---|---|
| 3,6 | 0,3 ml A + 0,1021 g B | 4,3 | 5,7 |
| | 3 ml A + 1,021 g B | 4,2 | 5,0 |
| | 7,5 ml A + 2,25 g B | 4,2 | 4,6 |
| | 15 ml A + 5,105 g B | 4,1 | 4,3 |
| Dispersion without buffer | 0 | 4.0 | 6,0 |
| Base material | 0 | n.a. | 5,8 |

| | | | |
|---|---|---|---|
| A = 0,2 M hydrochloric acid (HCL) B = potassium acid phthalate | | | |

### Results 2.2

The results from 1.2 show that also this buffer works well in lowering the elution pH, when the amount is sufficient enough. It is most likely that any buffer will have the same effect. Thus, any buffer can be used for this purpose, but preferred are the ones used in the examples.

### 3 Proton emitting acid groups:

### 3.1 Mixing acid groups to the dispersions

### 3.1.1. The effect on mixing Polyacrylic Acid to in a nonionic (NI) anionic (AI) surfactant stabilized vinyl acetate-co-ethylene (VA/E) copolymer dispersion.

| Concentration of polyacrylic acid | Formula (g/100 g 50 % dispersion) | Elution pH |
|---|---|---|
| Dispersion with 5 wt-% polyacrylic acid | 4,94 g A + 60,4 g B | 4,3 |
| Dispersion with 7 wt-% polyacrylic acid | 6,66 g A + 60,0 g B | 4,2 |
| Dispersion without polyacrylic acid | 0,0 g A + 62,0 g B | 5,2 |
| Base material | n.a. | 5,2 |

| | | |
|---|---|---|
| A = polyacrylic acid (35 wt-%) B = water | | |

### Results 3.1.1.

The results show that the effect on elution pH depending on the PAA mixed to the dispersion is OK.

### 3.1.2. The effect on elution pH of mixing the same amounts citric acid used in Examples 1.1. (Just citric acid and not in a buffer formulation).

| Corresponding pH buffer | Corresponding amount (g/100 g 50 % dispersion) | Buffer description | pH in dispersion | Elution pH |
|---|---|---|---|---|
| 2,6 | 0, 86 g A + 0, 86 g B | 2,6 a CA | 2,5 | 4,2 |
| | 4,3 g A + 4,3 g B | 2,6 b CA | 1,9 | 3,4 |
| 3,6 | 0,864 g A + 0, 864 g B | 3,6 a CA | 2, 6 | 4,4 |
| | 5,184 g A + 5,184 g B | 3, 6 b CA | 2,0 | 3,6 |
| 4,6 | 0,053 g A + 0, 053 g B | 4,6 a_CA | 2,7 | 4,4 |
| | 2,64 g A + 0,053 g B | 4, 6 b CA | 2,1 | 3,7 |
| Dispersion without buffer | 0 | | 4,0 | 6,0 |
| Base material | 0 | | | 6,0 |

| | | | | |
|---|---|---|---|---|
| A = citric acid B = water | | | | |

### Results 3.1.2

The elution pH is lowered also in this case but the efficiency is less and the results also show that the resulting pH is too low in most cases. A pH below 2 of the dispersions is not preferable, since they need special care during transport and handling.

### 3.2 Copolymerized acid groups to the base polymer. (High amounts of carboxylic acids are used to produce the latex by copolymerization with base acrylates and vinyl acetate.)

### 3.2.1 Dispersion compositions and properties.

| No | Polymer Composition (wt. %) | Surfactant Composition (pphm) | pH | Particle size (µm) | Glass transition (°C) |
|---|---|---|---|---|---|
| 201115 | VA/BA/AA 53/39/8 | K/L 0.8/2.5 | 3.8 | 0,27 | 9 |
| 201116 | VA/BA/AA 53/39/8 | K/M/N 0.8/1.9/0.6 | 3.8 | 0,19 | 11 |
| 201117 | VA/BA/AA 53/39/8 | L/O 2.4/0.8 | 4.7 | 0,29 | 8 |
| 201118 | VA/BA/AA 53/39/8 | L/0 0.8/2,3 | 3.8 | 0,2 | 9 |
| 201119 | MA/AA 95/5 | L/0 0.8/2,3 | 4.9 | 0,16 | 14 |
| 201120 | VA/BA/AA 49/36/15 | L/0 1.4/4.3 | 4.1 | 0,18 | 12 |

| Notation | Monomer name |
|---|---|
| VA | vinyl acetate |
| BA | butyl acrylate |
| AA | acrylic acid |
| MA | methyl acrylate |

| Notation | Surfactant name | Description | Supplier |
|---|---|---|---|
| K | Hostapur SAS 60 | secondary alkane sulphonate sodium salt | Clariant GmbH |
| L | Disponil A 3065 | fattyalcohol polylycolether, 30 EO | Cognis Deutschland GmbH&Co.KG |
| M | Disponil A 4080 | fattyalcohol polylycolether, 40 EO | Cognis Deutschland GmbH&Co.KG |
| N | Disponil A 1580 | fattyalcohol polylycolether, 15 EO | Cognis Deutschland GmbH&Co.KG |
| O | Disponil FES 77 IS | Fattyalcohol ethersulfate, 27 EO | Cognis Deutschland GmbH&Co.KG |

### 3.2.1 Polymerisation results.

By varying the amounts of copolymerized carboxylic acid as well as the base polymer and the surfactant system it was possible to show that we could produce dispersions having a large variation in composition and still being stable.

### VA/A-dispersions with different compositions of surfactants

| Sample number | pH in dispersion | Elution pH |
|---|---|---|
| 201115 | 3,8 | 4,5 |
| 201116 | 3,8 | 4,7 |
| 201117 | 4,7 | 4,5 |
| 201118 | 3,8 | 4,6 |
| 201119 | 4,9 | 5,6 |
| 201120 | 4,1 | 4,9 |
| Acrylic Dispersion without acid | 4,0 | 6,0 |
| Base material | | 6,0 |

### 3.2.1 Results.

By varying the amounts of copolymerized carboxylic acid as well as the base polymer and the surfactant system it was possible to show that we could produce dispersion that upon application also would lower the elution pH.

### 4 Bacteria test:

**Growth of bacteria in fibrous material**

| Sample description | E.coli | | | P. mirabilis | | | E. faecalis | | |
|---|---|---|---|---|---|---|---|---|---|
| | (10log cfu/ml added urine) | | | | | | | | |
| | 0h | 6h | 12h | 0h | 6h | 12h | 0h | 6h | 12h |
| Citrate-phosphate buffer (2,6d) | 3,5 | 3,3 | 3,3 | 3,5 | 2 | 2 | 3,4 | 2 | 2 |
| Citrate-phosphate buffer (3,6d) | 3,5 | 3,4 | 3,5 | 3,6 | <2 | <2 | 3,5 | 3,3 | 3,4 |
| Polyacrylic acid (7 wt-%) | 3,5 | 4,5 | 7,3 | 3,6 | 2,9 | 4,3 | 3,5 | 4 | 5,1 |
| | | | | | | | | | |
| Reference | 3,5 | 6,5 | 9,4 | 3,6 | 3,5 | 6,3 | 3,5 | 5 | 7,1 |

### 4 Results.

The most efficient hindrance of the bacterial growth is observed in the citrate-phosphate buffered system 2.6d. Where all the species of bacteria were affected. The efficiency is decreasing with increasing dispersion pH and it can also be seen that the efficiency for buffer is higher than added PAA. In all experiments there are large differences in the efficiency in bacterial hindrance between pH-regulated dispersions and the reference standard dispersion used.

## Claims

1. A binder composition for fibrous materials **characterised in that** it comprises a binder and a pH controlling agent which maintains the pH in an acidic range.

2. A binder composition according to claim 1, comprising a polymer dispersion as the binder.

3. A binder composition according to claim 1 or 2, wherein the pH controlling agent maintains the pH in a range of 1-6.

4. A binder composition according to claim 3, wherein the pH controlling agent maintains the pH in a range of 2-5.

5. A binder composition according to claim 4, wherein the pH controlling agent maintains the pH in a range of 3-4.

6. A binder composition according to any of the preceding claims, wherein said pH controlling agent is a buffer or a (poly)carboxylic acid.

7. A binder composition according to claim 6, wherein said buffer is selected from the group consisting of inorganic and organic buffers covering the pH-interval.

8. A binder composition according to claim 7, wherein said buffer is selected from the group consisting of phosphate-citrate buffer, citrate-citric acid buffer, phtalate-hydrochloric acid buffer.

9. A binder composition according to claim 6, wherein the (poly)carboxylic acid is polycarboxylic acid that is added to the binder.

10. A binder composition according to claim 6, wherein the (poly)carboxylic acid is carboxylic acid that is co-polymerized with the binder.

11. A binder composition according to any one of claims 6-10, wherein the (poly)carboxylic acid is selected from the group consisting of polyacids with a pKa below 6 suitable for addition to the dispersion to obtain the desired properties.

12. A binder composition according to any one of claims 6-11, wherein the (poly)carboxylic acid is selected from the group consisting of polyacrylic acid, polymethacrylic acid, polyitaconic acid.

13. A binder composition according to any one of the preceding claims, wherein the pH controlling agent is present in the binder composition in an amount of 0.1-10% by weight, based on dry matter.

14. A binder composition according to claim 13, wherein the pH controlling agent is present in the binder composition in an amount of 0.2-5% by weight, based on dry matter.

15. A binder composition according to claim 14, wherein the pH controlling agent is present in the binder composition in an amount of 0.3-4% by weight, based on dry matter.

16. A binder composition according to any one of claims 1-15, wherein the binder is a polymer dispersion comprising a polymer derived from at least one member selected from the group consisting of ethylene and vinyl containing compounds.

17. A binder composition according to claim 16, wherein the vinyl containing compounds include at least one unsaturated organic acid amide, at least one N-methylol derivative of at least one unsaturated organic acid amide and/or at least one ether of at least one N-methylol derivative of at least one unsaturated organic acid amide.

18. A binder composition according to claim 16, wherein the vinyl containing compounds are selected from the group consisting of acryl amide, N-methylolacrylamide, N-methylolmethacrylamide, N-(iso-butoxymethyl)-acrylamide and/or N-(n-butoxymethyl)acrylamide.

19. A binder composition according to claim 16, wherein the vinyl containing compounds are selected from the group consisting of glycidyl acrylate; glycidyl methacrylate; allyl methacrylate; di, tri and multifunctional ester of a di, tri or polyhydric alcohol and acrylic and/or methacrylic acid; trialkoxyvinylsilane; alkyldialkoxyvinylsilane; acryloxyalkoxysilane; acryloxyalkylalkoxysilane; alkoxyacrylsilane; methacryloxyalkoxysilane; methacryloxyalkylalkoxysilane and/or alkoxymethacrylsilane.

20. A binder composition according to claim 19, wherein the di, tri and multifunctional ester of a di, tri or polyhydric alcohol and acrylic and/or methacrylic acid is butanediol diacrylate, dipropylene glycol diacrylate, hexanediol diacrylate, tripropylene glycol diacrylate, butanediol dimethacrylate, ethylene glycol dimethacrylate, diethylene glycol methacrylate, trimethylolpropane triacrylate, trimethylolpropane triacrylate, trimethylolpropane triacrylate, ethoxylated trimethylolpropane tri acrylate and/or ethoxylated pentaerythritol diacrylate.

21. A binder composition according to claim 19, wherein said alkyl and/or alkoxy compounds are linear or branched having 1-4 carbon atoms, and most preferably are trimethoxyvinylsilane; triethoxyvinylsilane; triisopropoxyvinylsilane; methoxymethacrylsilane and/or 3-methacryloxypropyltriisopropoxysilane.

22. A binder composition according to any one of the preceding claims, wherein the binder is a polymer dispersion comprising a polymer derived from at least one member selected from the group consisting of ethylene and vinyl esters.

23. A binder composition according to claim 22, wherein the vinyl esters include vinyl acetate, vinyl propionate, vinyl butyrate, vinyl isobutyrate, vinyl 2-etylhexanoate, vinyl versatate and/or vinyl laurate.

24. A binder composition according to any one of claims 1-15, wherein the binder is a polymer dispersion comprising a polymer derived from at least one member selected from styrene acrylics.

25. A binder composition according to claim 24, wherein the styrene acrylics include copolymers of styrene and esters of acrylic and methacrylic acids.

26. A polymerisation process yielding a binder composition **characterised in that** the polymerisation process is performed in the presence of a pH controlling agent.

27. Use of a binder composition according to any one of claims 1-25 for providing antibacterial properties in a fibrous material.

28. Use according to claim 27, wherein the binder composition is effective against different bacteria families, such as E. coli, P. mirabilis and E. faecalis.

29. A fibrous material treated with a binder composition according to any one of the preceding claims, wherein the fibrous material comprise synthetic or natural fibers, or mixtures thereof.

30. A fibrous material according to claim 29, wherein the material comprise cellulosic fibers.

31. A fibrous material according to claim 29 or 30, wherein the material is non-woven materials.

32. A fibrous material according to any one of claims 29-31, wherein the binder composition is applied to the fibrous material by spraying, print bonding, saturation or impregnation.

33. A fibrous material according to any one of claims 29-32, wherein the binder composition is applied to the fibrous material in an amount of 1-50% (by weight).

34. A fibrous material according to claim 33, wherein the binder composition is applied to the fibrous material in an amount of 3-30% (by weight).

35. A fibrous material according to claim 34, wherein the binder composition is applied to the fibrous material in an amount of 5-25% (by weight).

36. A fibrous material according to claim 35, wherein the binder composition is applied to the fibrous material in an amount of 7-20% (by weight).

37. A fibrous material according to any one of claims 29-36, wherein the material is used for manufacturing diapers, incontinence protection products, female hygiene products, antibacterial packing material, sanitary and medical articles.
